# EUROPÄISCHE PATENTSCHRIFT

(11) **EP 3 877 009 B1**
(45) Veröffentlichungstag und Bekanntmachung des Hinweises auf die Patenterteilung: **01.10.2025**
(21) Anmeldenummer: 19703070.3
(22) Anmeldetag: 01.02.2019
(51) Int. Cl.: A61L 2/20, B01F 23/80, B65B 31/04

(54) **VERFAHREN ZUR KONSERVIERUNG EINER DISPERSION IN EINER DOSIERANLAGE UND DOSIERANLAGE**
PROCESS FOR PRESERVING A DISPERSION IN A METERING APPARATUS AND METERING APPARATUS
PROCÉDÉ DE CONSERVATION D'UNE DISPERSION DANS UN SYSTÈME DE DOSAGE ET SYSTÈME DE DOSAGE

(30) Priorität: 05.11.2018 DE 102018008666
(43) Veröffentlichungstag der Anmeldung: 15.09.2021
(73) Patentinhaber: Brillux GmbH & Co. KG, 48163 Münster (DE)
(72) Erfinder: HÖRSTING, Ingo, 48317 Drensteinfurt (DE); ERBER, Dietmar, 48143 Münster (DE); DRÜCKER, Ludger, 48153 Münster (DE); FELS, Sven, 48153 Münster (DE); STORB, Rainer, 48301 Nottuln (DE)
(74) Vertreter: Cohausz & Florack
(86) Internationale Anmeldenummer: PCT/EP2019/052537
(87) Internationale Veröffentlichungsnummer: WO 2020/094258

(56) Entgegenhaltungen:
- EP-A2- 1 189 995
- WO-A2-02/13774
- DE-A1- 19 942 284
- GB-A- 2 292 695
- US-A- 2 883 343
- US-A1- 2015 151 957

## Beschreibung

Die Erfindung betrifft ein Verfahren zur Konservierung einer Dispersionsfarbe oder einer Pigmentpaste in einer Dosieranlage und eine Dosieranlage zur Konservierung einer Dispersionsfarbe oder Pigmentpaste.

Dosieranlagen ermöglichen die präzise Bereitstellung einer bestimmten Menge einer Dispersion auf Abfrage. Wenn hier oder an anderer Stelle von Dosieranlagen die Rede ist, sind damit grundsätzlich sämtliche Systeme gemeint, die ein dosiertes Bereitstellen von einem Material ermöglichen. Dosieranlagen in diesem Sinne umfassen mindestens einen Behälter und ein schließbares Ventil.

Dosieranlagen finden beispielsweise in Farbmischanlagen Anwendung. Ein Beispiel für eine Dosieranlage für Farben ist in DE 196 54 829 A1 beschrieben.

Die in einer Dosieranlage gelagerte Dispersion verweilt zum Teil über mehrere Wochen und Monate in einem Behälter in der Dosieranlage. Damit sich während dieser Zeit die Qualität der Dispersion nicht durch mikrobiellen Befall durch Bakterien oder Pilze verschlechtert, wird der Dispersion Konservierungsmittel beigesetzt. Gängige Konservierungsmittel hierfür sind beispielsweise Isothiazoline oder Formaldehydabspalter. Die Menge an erforderlichem Konservierungsmittel übersteigt dabei die typisch tolerierbaren Konzentrationen in wässrigen Beschichtungssystemen.

Somit besteht ein Bedarf an Verfahren, die es ermöglichen, Dispersionen in Behältern von Dosieranlagen langfristig vor mikrobiellen Befall zu beschützen, ohne dabei auf Konservierungsmittel zurückgreifen zu müssen.

Als möglicher Ansatz hierfür wird in der EP 1 541 225 A1 eine Dosieranlage zum Mischen einer Dispersionsfarbe beschrieben, bei dem für jede Farbkomponente ein Behälter bereitgestellt wird, der über eine Förderleitung mit einem im Zufuhrbereich zu einem Mischgefäß angeordneten Dosierventil verbunden ist, und wobei die Behälter für die wässrigen Farbkomponenten durch wasser- und gasdichte Säcke gebildet sind. Das Innenvolumen der wasserdichten Säcke schrumpft beim Entleeren des Behälters entsprechend dem Volumen des Behälterinhalts, so dass kein Eintrocknen der Farbe an der Innenwand stattfindet und ein mikrobieller Befall im Gasraum oberhalb des Flüssigkeitsspiegels verhindert wird.

Allerdings kann die in EP 1 541 225 A1 beschriebene Dosieranlage nicht auf einfache Weise mit neuer Dispersionsfarbe aufgefüllt werden. Zudem kann bereits vorhandener mikrobieller Befall in den Säcken nicht mehr beseitigt werden und die Dosierförderung muss für eine genaue Dosierung permanent an den geänderten Innendruck in den Säcken angepasst werden. Des Weiteren lassen sich die in der EP 1 541 225 A1 beschriebenen Säcke nicht auf einfache Weise an ein bereits bestehendes Farbmischsystem oder ähnliches anbringen und müssen nach der Verwendung entsorgt werden, wodurch Verpackungsmüll entsteht.

Die Druckschrift GB 2 292 695 A offenbart ein Verfahren zur Herstellung einer Acrylemulsionsfarbe. Dabei wird das Acrylemulsionsharz aus einem von zwei Vorratsbehältern in einen von zwei Mischbehältern geleert und mit einem Rührwerk gemischt. Pigmente, Verdickungsmittel, Dispergiermittel, Extender und pH-Einsteller werden in einem Dispersionsbehälter in einer Wasserbasis dispergiert und die Pigmentdispersion wird in das Harz in dem Mischbehälter eingebracht.

Die Druckschrift US 2 883 343 A offenbart ein Verfahren und eine Vorrichtung zur Analyse eines Schmierölbestandes, der einen Zusatzstoff enthält, und ein Verfahren und eine Vorrichtung zur Kontrolle der Vermischung von Zusatzstoffen mit Schmierölbeständen.

Die Druckschrift EP 1 189 995 A2 beschreibt eine konservierte Farbzusammensetzung auf Wasserbasis, die eine Oxidoreduktase, ein Oxidationsmittel, ein Bindemittel und mindestens 10 Gew.-% Wasser enthält.

Die Druckschrift US 2015/151957 A1 beschreibt einen Wasserspender der einen Kaltwassertank, eine Frischwasserleitung, einen austauschbaren Frischwasserbehälter, eine Pumpe, eine Luftansaugleitung und einen Ozongenerator, der mit der Luftansaugleitung in Verbindung steht, sowie ein Steuergerät, das so konfiguriert ist, dass es den Ozongenerator aktiviert, aufweist.

Die Druckschrift DE 199 42 284 A1 beschreibt eine Vorrichtung zur Herstellung von flüssigen Präparationen mit mindestens einem gasförmigen volatilen Anästhesiemittel, wobei die Vorrichtung eine Vormischstufe und eine nachgeschaltete Feinmisch- oder Homogenisierungsstufe enthält.

Die Druckschrift WO 02/13774 A2 beschreibt ein Verfahren zur Herstellung einer wässrigen Suspension von Calciumcarbonat zur Verwendung bei der Herstellung von Zahnpastazusammensetzungen, umfassend die Zugabe eines Dispersionsmittels und eines Konservierungsmittels zu einer Suspension von Calciumcarbonat und die Zugabe von Wasser zu der Suspension, wobei das Wasser durch ein Verfahren zur Verringerung der mikrobiologischen Aktivität darin behandelt wurde.

Die vorliegende Erfindung stellt sich somit die Aufgabe, ein Verfahren bereitzustellen, bei dem konservierungsmittelfreie oder konservierungsmittelarme Dispersionsfarben oder Pigmentpasten in Behältern von Dosieranlagen langfristig vor mikrobiellen Befall geschützt werden.

Zudem stellt sich die Erfindung zur Aufgabe, ein besonders nachhaltiges Verfahren zur Konservierung von Dispersionsfarben oder Pigmentpasten in Behältern von Dosieranlagen bereitzustellen, ohne dabei auf herkömmliche Konservierungsmittel, insbesondere Isothiazolinone, zurückgreifen zu müssen.

Eine weitere Aufgabe der vorliegenden Erfindung ist es, ein Verfahren zu ermöglichen, das sich flexibel auf bereits existierende Dosieranlagen und/oder Farbmischanlagen anwenden lässt.

Des Weiteren stellt sich die Erfindung die Aufgabe, eine Dosieranlage zur Konservierung einer Dispersionsfarbe oder Pigmentpaste bereitzustellen, in der eine Dispersionsfarbe oder Pigmentpaste langfristig lagerbar ist, ohne der Gefahr eines mikrobiellen Befalls ausgesetzt zu sein.

Weitere Aufgaben ergeben sich aus den nachfolgenden Ausführungen und werden teilweise hiernach aufgeführt.

Alle oder einige dieser Aufgaben werden erfindungsgemäß durch das Verfahren nach Anspruch 1 und die Dosieranlage nach Anspruch 8 gelöst.

Vorteilhafte Ausgestaltungen der Erfindung sind in den abhängigen Ansprüchen angegeben und werden nachfolgend im Einzelnen erläutert.

Gemäß dem erfindungsgemäßen Verfahren wird zur Konservierung einer Dispersionsfarbe oder Pigmentpaste in einem Behälter, der Teil einer Dosieranlage ist, ein gasförmiges Oxidationsmittel in den Behälter eingebracht, wobei das gasförmige Oxidationsmittel in den Gasraum über der Oberfläche der Dispersionsfarbe oder Pigmentpaste in den Behälter eingebracht wird. Vorzugsweise wird die Dispersionsfarbe oder Pigmentpaste nach dem Einbringen in den Behälter durchmischt, z.B. durch Rühren.

Überraschend hat sich gezeigt, dass mit einem Verfahren gemäß Anspruch 1, eine Dispersionsfarbe oder Pigmentpaste über Monate hinweg vor mikrobiellen Befall geschützt werden kann. Die Dispersionsfarbe oder Pigmentpaste kann hierbei frei von Konservierungsmitteln sein und weist trotzdem einen langanhaltenden Schutz gegen mikrobiellen Befall auf. Selbst bei häufiger Benutzung der Dosieranlage und einem wiederholten Auffüllen des Behälters mit frischer Dispersionsfarbe oder Pigmentpaste wird durch das erfindungsgemäße Verfahren ein Schutz vor Pilz- und Bakterienbefall gewährleistet.

Ohne an eine bestimmte wissenschaftliche Theorie gebunden sein zu wollen, scheint das in den Behälter eingebrachte Oxidationsmittel für einen vollständigen Schutz vor mikrobiellem Befall zu sorgen, indem Mikroben wie Pilze und Bakterien durch das Oxidationsmittel abgetötet werden.

Das Oxidationsmittel bietet der Dispersionsfarbe oder Pigmentpaste einen langanhaltenden Schutz vor mikrobiellem Befall. Insbesondere bei häufiger Entnahme der Dispersionsfarbe oder Pigmentpaste aus dem Behälter der Dosieranlage kann es sinnvoll sein, dass neues Oxidationsmittel in den Behälter eingebracht wird, um den Schutz vor mikrobiellem Befall zu erneuern. Gemäß einer bevorzugten Ausführungsform der Erfindung wird das Oxidationsmittel in periodischen Zeitabständen, vorzugsweise mindestens einmal pro Monat oder mindestens zweimal pro Monat, weiter bevorzugt mindestens dreimal pro Monat, noch bevorzugter mindestens einmal pro Woche, am bevorzugtesten einmal pro Tag, in den Behälter eingebracht. Durch das Einbringen des Oxidationsmittels in den Behälter in periodischen Zeitabständen behält die Dispersionsfarbe oder Pigmentpaste besonders lange einen Schutz vor mikrobiellem Befall. Wird das Oxidationsmittel in periodischen Zeitabständen mindestens einmal pro Tag eingebracht, so kann die Dispersionsfarbe oder Pigmentpaste über mehrere Jahre Schutz vor Pilz- und Bakterienbefall behalten.

Zweckmäßigerweise ist vorgesehen, dass pro Einbringung 0,1 bis 200 mg, vorzugsweise 0,5 bis 100 mg oder besonders bevorzugt 1 bis 50 mg gerechnet pro Liter Behältervolumen des Oxidationsmittels eingebracht werden. Es hat sich herausgestellt, dass mit diesen Mengen ein besonders gutes Gleichgewicht zwischen effektiver Wirkung des Konservierungsschutzes durch das Oxidationsmittel und möglichst geringem Verbrauch an Ressourcen gelingt. Ferner kann so bei gasförmigen Oxidationsmitteln eine effiziente Prozessführung erreicht werden. Beispielsweise kann bei Verwendung von Ozon als Oxidationsmittel, das mit einem Ozongenerator mit einer Generatorleistung von 500 mg/h erzeugt wird, für eine Anlage umfassend zwanzig eine Dispersionsfarbe oder Pigmentpaste enthaltende Behälter innerhalb von 5 Minuten eine effektive Menge an Oxidationsmittel in die Behälter eingebracht werden.

Erfindungsgemäß wird das Oxidationsmittel in den Gasraum über der Oberfläche der Dispersionsfarbe oder Pigmentpaste in den Behälter eingebracht. Dadurch gelingt es, den besonders problematischen Bereich für mikrobiellen Befall im Gasraum über der Oberfläche der Dispersionsfarbe oder Pigmentpaste effektiv zu konservieren.

Grundsätzlich eignen sich ganz unterschiedliche Oxidationsmittel zur Konservierung der Dispersion.

Vorzugsweise weist das Oxidationsmittel mit Bezug auf die Normalwasserstoffelektrode bei einer Temperatur von 25°C und einer effektiven Konzentration von 1 mol l⁻¹ und/oder einer Ionenaktivität von 1 oder bei gasförmigen Reaktanden bei einem Partialdruck von 101,325 kPa ein Normalpotential von 0,1 V oder höher, bevorzugt 0,5 V oder höher, weiter bevorzugt 1 V oder höher, auf. Stärkere Oxidationsmittel bekämpfen Mikrobenbefall effizienter. Insbesondere sind Isothiazoline keine Oxidationsmittel im Sinne der Erfindung.

Vorteilhafterweise ist das Oxidationsmittel ein sauerstoff- oder chlorbasiertes Oxidationsmittel oder eine Mischung davon, bevorzugt ein sauerstoff-basiertes Oxidationsmittel. Vorzugsweise wird das Oxidationsmittel ausgewählt aus der Gruppe bestehend aus Natriumhypochlorit, Kaliumhypochlorit, Javelwasser, Chlor, Ozon, Wasserstoffperoxid, Peroxyessigsäure, Perborat, Percarbonat, und Mischungen davon. Diese Oxidationsmittel stellen starke Oxidationsmittel dar, die das Wachstum von mikrobiellen Organismen effektiv unterbinden.

Besonders bevorzugt wird das Oxidationsmittel ausgewählt aus der Gruppe bestehend aus Natriumhypochlorit, Wasserstoffperoxid, Ozon, und Mischungen davon. Die Oxidationsmittel aus der genannten Gruppe haben sich als besonders gut geeignet für den Schutz der Dispersionsfarbe oder Pigmentpaste vor mikrobiellem Befall herausgestellt, da sie effektive Oxidationsmittel darstellen. Ferner sind diese Oxidationsmittel insbesondere bei den zweckmäßigerweise vorgesehenen Mengen für die Dispersionsfarbe oder Pigmentpaste im Wesentlichen unproblematisch. Insbesondere sorgen die genannten Oxidationsmittel bei den zweckmäßigerweise vorgesehenen Mengen nicht zu einer nennenswerten Veränderung der Farbe und/oder Qualität der Dispersionsfarbe oder Pigmentpaste.

Erfindungsgemäß ist das Oxidationsmittel gasförmig. Gasförmige Oxidationsmittel lassen sich auf einfache Weise in den Behälter leiten und erzeugen eine Schutzatmosphäre im Gasraum über der Oberfläche der Dispersionsfarbe oder Pigmentpaste. Dies führt zu einem besonders lang anhaltenden Schutz der Dispersionsfarbe oder Pigmentpaste vor mikrobiellem Befall. Die Zugabe eines gasförmigen Oxidationsmittels ist besonders vorteilhaft, da auch über lange Standzeiten einer Dispersionscharge allenfalls geringe Verdünnungseffekte auftreten.

Gemäß einer besonders bevorzugten Ausführungsform der Erfindung ist das Oxidationsmittel Ozon. Wird Ozon als Oxidationsmittel im erfindungsgemäßen Verfahren eingesetzt, erfolgt eine besonders langanhaltende antimikrobielle Wirkung. Ozon ist zudem insbesondere in den zweckmäßigerweise vorgesehenen Mengenzusätzen mit den gängigen Bestandteilen von Dispersionsfarben oder Pigmentpasten kompatibel. Zudem lässt sich Ozon sehr leicht am Ort der Verwendung herstellen.

Das Ozon kann dabei auf unterschiedliche Weise erzeugt werden. Vorzugsweise kann das Ozon in einem Generator oder im Deckel des Behälters, insbesondere mittels Koronaentladung, erzeugt werden.

Das Ozon kann extern, das heißt außerhalb des Behälters, der Teil einer Dosieranlage ist, erzeugt werden und anschließend in den Behälter eingebracht werden. Dabei kann das Ozon beispielsweise aus einer chemischen Reaktion von Kaliumpermanganat mit konzentrierter Schwefelsäure erzeugt werden oder durch Elektrolyse von verdünnter Schwefelsäure, insbesondere bei tiefen Temperaturen. Ozon kann auch aus Luft oder Sauerstoff unter Einwirkung von UV-Strahlung erzeugt werden.

Gemäß einer bevorzugten Ausführungsform des erfindungsgemäßen Verfahrens wird das Ozon in einem Ozongenerator umfassend eine Spannungsquelle, insbesondere einen Hochspannungsgenerator, und eine Entladungseinheit ausgehend von Luft, insbesondere getrockneter Luft, Sauerstoff oder einem Sauerstoffgemisch mit Argon oder Kohlenstoffdioxid erzeugt. Der Aufbau gängiger Ozongeneratoren ist dem Fachmann bekannt. In der Entladungseinheit werden Sauerstoffmoleküle vorzugsweise durch stille elektrische Entladung, sogenannte "*Koronaentladung*", zu Sauerstoffatomen dissoziiert, wonach noch im Plasma der Entladungsfilamente die Ozonsynthese und Ozonanreicherung stattfindet. Dabei kann der entstehende Ozonanteil an der Endkonzentration des Gasgemisches bei Luft als Ausgangsgas 1 bis 5 Gew.% und bei Sauerstoff als Ausgangsgas 6 bis 13 Gew.% betragen. Vorzugsweise wird Luft als Ausgangsgas zur Ozonerzeugung eingesetzt. Bei der Erzeugung des Ozons in einem Generator wird somit ausgehend von günstigen Startmaterialien auf saubere Weise Ozon hergestellt.

Ein möglicher Aufbau einer als Ozonisator bezeichneten Entladungseinheit, die sich für das erfindungsgemäße Verfahren zur Erzeugung des Ozons eignet, ist in DE 197 14 176 A1 beschrieben.

Gemäß einer bevorzugten Ausführungsform der Erfindung wird das Ozon im Deckel des Behälters erzeugt. Das Erzeugen des Ozons im Deckel des Behälters hat den wesentlichen Vorteil, dass das Ozon einen kürzeren Weg zurücklegen muss, um zur Dispersionsfarbe oder Pigmentpaste und somit zum Wirkungsort zu gelangen. Durch diese Ausführungsform wird sichergestellt, dass möglichst wenig erzeugtes Ozon aufgrund der Instabilität des Ozons verloren geht. Zudem ist die Erzeugung des Ozons im Deckel des Behälters ein besonders platzsparender und flexibler Ansatz.

Das Erzeugen des Ozons im Deckel des Behälters kann gemäß einer Ausführungsform des erfinderischen Verfahrens durch eine im Deckel des Behälters angeordnete Vorrichtung erfolgen, indem durch UV-Licht Ozon aus Luft oder Sauerstoff erzeugt wird. Hierfür umfasst der Deckel beispielsweise UV-C Leuchtdioden. Gemäß einer bestimmten Ausführungsform dieses Ansatzes weist der Deckel eine Induktionsplatte, eine Kontroll-LED, mehrere UV-C LED, vorzugsweise von 2 bis 8 LED, weiter bevorzugt von 3 bis 5 LED, mehrere Blaulicht LED, einen Akku und einen Ein- und Ausschalter auf. Allerdings sind auch Deckel mit einem anderen spezifischen Aufbau als Deckel des Behälters für die Erzeugung von Ozon durch UV-Strahlung geeignet.

Gemäß einer bevorzugteren Ausführungsform der Erfindung wird das Ozon im Deckel des Behälters durch Koronaentladung erzeugt. Bevorzugt passiert hierbei Luft oder Sauerstoff eine Röhre, die über einen Adapter direkt am Deckel des Behälters angebracht ist und eine Metallfolie, einen Metallstab und einen Hohlraum umfasst. Die Luft passiert den Hohlraum während Metallfolie und Metallstab unter Strom gesetzt werden und zur Koronaentladung führen. Auf diese Weise wird in der Röhre Ozon erzeugt, das direkt in den Behälter tritt. Diese Ausführungsform erzeugt einen besonders effizienten Schutz der Dispersionsfarbe oder Pigmentpaste vor mikrobiellem Befall.

Bei einer weiteren bevorzugten Ausführungsform wird Ozon durch Koronaentladung außerhalb des Behälters, jedoch in unmittelbarer Nähe des Behälters, erzeugt. Bei dieser Ausführungsform wird das Ozon vorzugsweise mittels einer Pumpe, insbesondere einer Membranpumpe, in den mindestens einen Behälter eingebracht.

Vorzugsweise wird das Ozon gleichmäßig in den Behälter eingebracht. Dabei erzeugt die Pumpe zweckmäßigerweise einen Überdruck. Die Pumpe und die Behälter sind vorzugsweise über die Zuleitung, beispielsweise ein Schlauchsystem, verbunden. So ist sichergestellt, dass bei Verwendung eines Ozongenerators in unmittelbarer Nähe der Behälter alle Behälter mit ausreichender Menge Ozon versorgt werden können.

Vorzugsweise wird die Dispersionsfarbe oder Pigmentpaste nach dem Einbringen des Oxidationsmittels durchmischt. Gemäß einer bevorzugten Ausführungsform des erfinderischen Verfahrens wird die Dispersionsfarbe oder Pigmentpaste nach dem Einbringen des Oxidationsmittels durch Rühren durchmischt. Auf diese Weise wird der durch das Oxidationsmittel bereitgestellte antimikrobielle Schutz in der gesamten Dispersionsfarbe oder Pigmentpaste verteilt. Zudem sorgt das Durchmischen der Dispersionsfarbe oder Pigmentpaste für ein stabiles, homogenes Erscheinungsbild der Dispersionsfarbe oder Pigmentpaste.

Pigmentpasten sind beispielsweise in EP 2 243 808 B1 beschrieben. Dispersionsfarben und Pigmentpasten sind besonders geeignet für das erfindungsgemäße Verfahren, da sie zum einen besonders gut durch das Oxidationsmittel vor mikrobiellem Befall geschützt werden können und zum anderen weil sie von besonderer wirtschaftlicher Bedeutung sind. Handelt es sich um eine Pigmentpaste, kann sie beispielsweise zum Abtönen von Farben, vorzugsweise Dispersionsfarben, verwendet werden. Pigmentpasten enthalten vorzugsweise keine Bindemittel wie Polymerdispersionen.

Gemäß einer besonders bevorzugten Ausführungsform der Erfindung ist die Dispersionsfarbe oder Pigmentpaste im Wesentlichen frei von Konservierungsmitteln. "Im Wesentlichen frei von Konservierungsmitteln" bedeutet dabei, dass die Pigmentpaste Konservierungsmittel höchstens in Spuren, insbesondere in einer Menge von weniger als 2 ppm enthält.

In den Druckschriften EP 1 297 079 B1, DE 1 031 910, DE 10 2014 013 455 A1 und DE 10 2016 002 221 A1 werden jeweils Dispersionsfarben beschrieben, die im Wesentlichen frei von Konservierungsmitteln sind. Die dort beschriebenen Dispersionsfarben eignen sich hervorragend für das erfindungsgemäße Verfahren.

Ein weiterer Aspekt der Erfindung betrifft zudem eine Dosieranlage.

Die erfindungsgemäße Dosieranlage zur Konservierung einer Dispersionsfarbe oder Pigmentpaste umfasst mindestens einen Behälter, der zur Lagerung einer Dispersionsfarbe oder Pigmentpaste ausgelegt ist und einen Höchstfüllstand für die Dispersionsfarbe oder Pigmentpaste aufweist, ein schließbares Dosierventil sowie mindestens eine in den Behälter führende Zuleitung für ein gasförmiges Oxidationsmittel, wobei die in den Behälter führende Zuleitung oberhalb eines Höchstfüllstandes in den Behälter führt.

Die erfindungsgemäße Dosieranlage eignet sich hervorragend zum antimikrobiellen Schutz der Dispersionsfarbe oder Pigmentpaste und für das Durchführen des erfindungsgemäßen Verfahrens.

Der Behälter der erfindungsgemäßen Dosieranlage weist, gemäß einer bevorzugten Ausführungsform der Erfindung, Mittel zum Durchmischen einer Dispersionsfarbe oder Pigmentpaste auf. Auf diese Weise wird der durch das Oxidationsmittel bereitgestellte antimikrobielle Schutz in der gesamten Dispersionsfarbe oder Pigmentpaste verteilt. Zudem sorgt das Durchmischen der Dispersionsfarbe oder Pigmentpaste für ein stabiles, homogenes Erscheinungsbild der Dispersionsfarbe oder Pigmentpaste. Vorzugsweise weist der Behälter als Mittel zum Durchmischen ein Rührwerk auf. Ein Rührwerk eignet sich besonders gut zur effektiven Durchmischung der Dispersionsfarbe oder Pigmentpaste.

Die in den Behälter führende Zuleitung kann auf verschiedenen Höhen in den Behälter führen. Erfindungsgemäß führt die in den Behälter führende Zuleitung oberhalb des Höchstfüllstands des Behälters in den Behälter. Dadurch gelingt es, den besonders problematischen Bereich für mikrobiellen Befall im Gasraum über der Oberfläche der Dispersionsfarbe oder Pigmentpaste, effektiv zu konservieren. Ferner ist der Konstruktionsaufwand der Zuleitung geringer, da ein Eindringen der Dispersionsfarbe oder Pigmentpaste in die Zuleitung nicht vermieden werden muss.

Vorzugsweise endet die in den Behälter führende Zuleitung in einer Düse. Dadurch kann das Oxidationsmittel effektiv in den Behälter oder direkt in die Dispersionsfarbe oder Pigmentpaste eingebracht werden, je nachdem auf welcher Höhe die Zuleitung in den Behälter führt. Vorzugsweise ist die Düse in Richtung des Behälterbodens gerichtet. Durch diese Düsenanordnung wird eine Verschmutzung der Düse beim Befüllen des Behälters mit neuer Dispersionsfarbe oder Pigmentpaste vermieden. Gemäß einer alternativen bevorzugten Ausführungsform kann die in den Behälter führende Zuleitung an ihrer Austrittsöffnung mit einer in Richtung des Behälterbodens geöffneten Schutzkappe versehen sein. Durch die Schutzkappe wird eine Verschmutzung und ein daraus resultierendes Verstopfen der Austrittsöffnung beim Befüllen des Behälters mit Dispersionsfarbe oder Pigmentpaste vermieden.

Gemäß einer Ausführungsform führt die in den Behälter führende Zuleitung in den Behälter oberhalb seines Höchstfüllstands und endet in einer Düse.

Vorzugsweise erfolgt die Einleitung des Oxidationsmittels in den Innenraum des Behälters über einen Adapter, der an seiner Oberseite mit dem Deckelteil des Behälters und an seiner Unterseite mit der Seitenwand des Behälters im Wesentlichen dicht abschließt, z.B. in Form eines Verlängerungsstutzens für ein Rohr. Der Adapter kann als Bestandteil des Behälterdeckels ausgestaltet sein. Der Adapter umfasst eine Zuleitung mit einer Austrittsöffnung, über die das Oxidationsmittel in den Innenraum des Behälters geleitet werden kann. Die Austrittsöffnung kann als Düse ausgestaltet sein. Gemäß einer bevorzugten Ausführungsform der Erfindung befindet sich an der Austrittsöffnung im Adapter eine in Richtung des Behälterbodens geöffnete Schutzkappe. Durch die Schutzkappe wird eine Verschmutzung und ein daraus resultierendes Verstopfen der Austrittsöffnung beim Befüllen des Behälters mit neuer Dispersionsfarbe oder Pigmentpaste vermieden. Geeignete Adapter sind beispielsweise in den

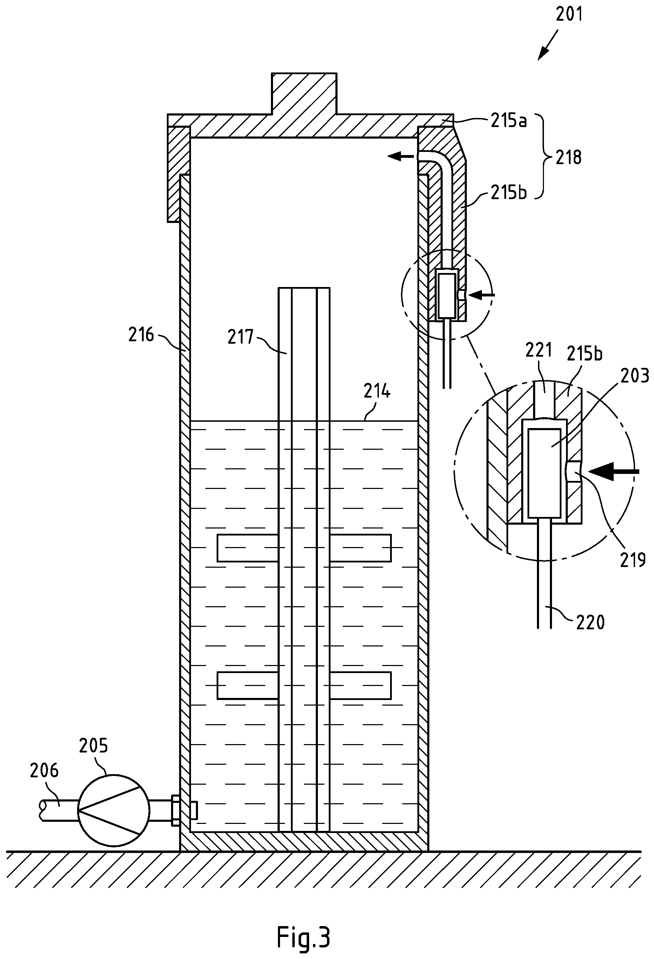

Figuren 3 bis 8 und den zugehörigen Figurenbeschreibungen beschrieben. Während die in den Figuren 3 bis 8 dargestellten Adapter für den Betrieb mit Ozon als Oxidationsmittel optimiert sind, können diese auch mit beliebigen anderen flüssigen oder gasförmigen Oxidationsmitteln betrieben werden. Der Vorteil der Verwendung von Adaptern besteht darin, dass bestehende Dosieranlagen auf einfache Weise und ohne aufwändige Maßnahmen (wie z.B. das Bohren von Löchern in die Behälterwandungen für die Zuleitung des Oxidationsmittels) für den Betrieb mit dem erfindungsgemäßen Verfahren umgerüstet werden können.

Denkbar ist ferner, dass der mindestens eine Behälter mehr als eine in den Behälter führende Zuleitung aufweist. Beispielsweise kann der Behälter zwei oder mehr in den Behälter führende Zuleitungen aufweisen. Die Zuleitungen können auf derselben Höhe in den Behälter führen, oberhalb des Höchstfüllstands. Dies führt zu einer gleichmäßigeren Verteilung. Mehrere Zuleitungen pro Behälter können aber auch auf verschiedenen Höhen in den Behälter führen, beispielsweise oberhalb und unterhalb des Höchstfüllstands. Dadurch kann ein direktes Einbringen des Oxidationsmittels in die Dispersionsfarbe oder Pigmentpaste sowie eine Behandlung des Behälters oberhalb des Höchstfüllstands erreicht werden. Die Zuleitungen können ferner jeweils in einer Düse enden.

Der mindestens eine Behälter ist, gemäß einer bevorzugten Ausführungsform der Erfindung, aus Kunststoff. Kunststoffe eignen sich gut als Material für Behälter, da sie gegenüber vielen Oxidationsmitteln beständig sind. Der Behälter ist vorzugsweise aus Polyoxymethylen (POM), Polypropylen, Polyethylen, Polyethylenterephthalat, Polyamid oder Mischungen oder Blends davon. Diese Kunststoffe haben sich als besonders beständig und langlebig herausgestellt. Neben den genannten Kunststoffen kann auch Edelstahl als Material für den mindestens einen Behälter verwendet werden.

Der mindestens eine Behälter kann ganz unterschiedliche Formen aufweisen. Gemäß einer möglichen Ausführungsform der Erfindung ist der mindestens eine Behälter quaderförmig. Gemäß einer bevorzugten Ausführungsform der Erfindung ist der Behälter im Wesentlichen zylinderförmig. Bei einer im Wesentlichen zylinderförmigen Ausgestaltung des Behälters werden Ablagerungen in Ecken oder Kanten des Behälters vermieden und der Schutz vor mikrobiellem Befall verstärkt.

Gemäß einer weiteren Ausführungsform der Erfindung weist die Dosieranlage mindestens zwei, vorzugsweise mindestens drei oder mindestens vier Behälter auf. Die Behälter können unterschiedliche Dispersionsfarben oder Pigmentpasten enthalten und über eine gemeinsame Steuereinrichtung gesteuert werden. Auf diese Weise können verschiedene Dispersionsfarben oder Pigmentpasten ausgehend von einem Steuersystem gehandhabt werden und die Dispersionsfarben oder Pigmentpasten präzise miteinander gemischt werden.

Weist die Dosieranlage mehrere Behälter auf, so können die Behälter karussellartig oder auch stationär angeordnet sein. Vorzugsweise sind die mehreren Behälter karussellartig angeordnet. Auf einem Karussell können dann in zentraler Achse konzentrisch ein oder mehrere Eimer zum Aufnehmen der Dispersionsfarben oder Pigmentpasten vorliegen. Bei dieser Ausführungsform können die Dispersionsfarben oder Pigmentpasten der mehreren Behälter besonders gut miteinander gemischt werden.

Gemäß einer weiteren bevorzugten Ausführungsform der Erfindung ist die Zuleitung aus einem Material enthaltend mindestens einen Kunststoff ausgewählt aus der Gruppe bestehend aus Polyurethan (PUR), Polytetrafluoroethylen (PTFE), Perfluoralkoxy-Polymer (PFA), Polyvinylidenfluorid, Perfluorkatschuk, Ethylen-Tetrafluorethylen, Tetrafluorethylen-Hexafluorpropylen-Copolymer, Ethylen-Chlortrifluorethylen, Ethylen-Propylen-Dien-Kautschuk, und Mischungen davon. Das Material der Zuleitung kann auch aus einem der vorgenannten Kunststoffe bestehen. Materialien enthaltend oder bestehend aus den vorgenannten Kunststoffen zeichnen sich durch eine hohe Stabilität gegenüber starken Oxidationsmitteln aus. Eine Zuleitung aus diesen Materialien weist somit eine hohe Lebensdauer und ein hohes Maß an Flexibilität auf. Insbesondere ist eine Zuleitung aus den genannten Materialien zum Zuführen von Wasserstoffperoxid, Natriumhypochloritlösungen und/oder Ozon geeignet.

Die Zuleitung kann beispielsweise einen oder mehrere Schläuche umfassen oder daraus bestehen. Die Zuleitung kann durch ein Schlauchsystem gebildet werden.

Enthält die Dosieranlage mehr als einen Behälter, kann die Dosieranlage auch mehr als eine in den Behälter führende Zuleitung aufweisen. Vorzugsweise weist jeder Behälter der Dosieranlage, der zur Lagerung einer Dispersionsfarbe oder Pigmentpaste ausgelegt ist, eine in den Behälter führende Zuleitung auf.

Die erfindungsgemäße Dosieranlage umfasst gemäß einer bevorzugten Ausführungsform einen Ozongenerator. Vorzugsweise umfasst dabei der Ozongenerator eine Spannungsquelle, insbesondere einen Hochspannungsgenerator, und eine Entladungseinheit. Bevorzugt wird in der Entladungseinheit mittels Koronaentladung Ozon erzeugt. Diese Form der Ozonerzeugung hat sich als besonders effizient herausgestellt.

Gemäß einer Ausführungsform der Erfindung sind die Spannungsquelle, insbesondere der Hochspannungsgenerator, und die Entladungseinheit im selben Gerätegehäuse angeordnet. Dadurch ist es möglich, dass die Koronaentladung an einem Ort zentral in der Dosieranlage und damit räumlich getrennt von dem mindestens einen Behälter stattfindet. Somit kann an einem Ort zentral in der Dosieranlage Ozon erzeugt werden. Anschließend kann das zentral erzeugte Ozon in der Dosieranlage verteilt werden, vorzugsweise über die in den mindestens einen Behälter führende Zuleitung. Diese Ausführungsform umfasst in der Regel mindestens einen Ozongenerator und optional mindestens eine Pumpe, insbesondere eine Membranpumpe, zum Transport des Ozons in den mindestens einen Behälter, vorzugsweise über die Zuleitung. Mit Hilfe der Pumpe wird der mindestens eine Behälter über die Zuleitung mit Ozon versorgt. Die Pumpe erzeugt vorzugsweise einen Überdruck. Die Pumpe versorgt den mindestens einen Behälter vorteilhafterweise gleichmäßig mit dem erzeugten Ozon.

Mit der optionalen Pumpe kann sichergestellt werden, dass bei einer Dosieranlage mit mehr als einem Behälter und bei Verwendung eines Ozongenerators in unmittelbarer Nähe der Behälter alle Behälter mit ausreichender Menge Ozon versorgt werden können.

Gemäß einer weiteren Ausführungsform der Erfindung sind die Spannungsquelle, insbesondere der Hochspannungsgenerator, und die Entladungseinheit nicht im selben Gerätegehäuse angeordnet. Vorzugsweise ist die Entladungseinheit im Behälterdeckel, bevorzugt in einem Adapter des Behälterdeckels. Gemäß dieser Ausführungsform der Erfindung wird Ozon vorzugsweise mittels Koronaentladung in den jeweiligen Behälterdeckeln erzeugt. Die Behälterdeckel weisen hierfür vorzugsweise einen Deckelteil und einen Adapter auf. In diesen Adaptern wird das Ozon dann mittels Koronaentladung erzeugt. Auf diese Weise wird der Weg, den das *in situ* erzeugte Ozon zurücklegen muss, minimiert und somit die Menge an für die Konservierung der Dispersionsfarbe oder Pigmentpaste zur Verfügung stehendem, reaktivem Ozon maximiert.

Für die Herstellung des Ozons gilt dabei das vorstehend im Zusammenhang mit dem erfindungsgemäßen Verfahren Gesagte entsprechend.

Die Dosieranlage ist, gemäß einer besonders bevorzugten Ausführungsform der Erfindung, zur präzisen Dosierung der Dispersionsfarbe oder Pigmentpaste mit einer Steuereinrichtung, vorzugsweise einem Computer, verbunden.

Die Dosieranlage kann ferner ein Dosierventil umfassen. Vorzugsweise ist das schließbare Ventil ein Dosierventil. Weiterhin kann die Dosieranlage eine Förderleitung umfassen. Die Dosieranlage kann auch eine Förderpumpe umfassen. Mit der Förderpumpe kann die Dispersionsfarbe oder Pigmentpaste in die Förderleitung eingebracht werden. Das Dosierventil ist bevorzugt an der Förderleitung angebracht. Umfasst die Dosieranlage ein Dosierventil, ist das Dosierventil bevorzugt mit der Steuereinrichtung verbunden. Durch die Förderleitung kann die Dispersionsfarbe oder Pigmentpaste mithilfe einer Förderpumpe, die vorzugsweise auch mit der Steuereinrichtung verbunden ist, vom Behälter, vorzugsweise über einen Füllkopf, auf eine Waage bzw. in einen Eimer, der auf der Waage aufliegt, gebracht werden. Bevorzugterweise ist die Waage ebenfalls mit der Steuereinrichtung verbunden, um eine Steuerung der Förderpumpe und des Dosierventils in Abhängigkeit der gewogenen Menge an Dispersionsfarbe oder Pigmentpaste zu ermöglichen. Neben der gravimetrischen Dosierung kann die Mengendefinierung alternativ auch volumetrisch über Pumpen, insbesondere Dosierpumpen wie Zahnradpumpen, Hubkolbenpumpen, Exzenterschneckenpumpen oder Membranpumpen, erfolgen. Vorteilhaft an dieser Ausführungsform ist, dass auf eine Waage gänzlich verzichtet werden kann.

Das beschriebene System einer Dosieranlage ermöglicht eine besonders präzise Dosierung der Dispersionsfarbe oder Pigmentpaste, die flexibel einsetzbar ist.

Um eine gute Durchmischung der Dispersionsfarbe oder Pigmentpaste im Eimer auf der Waage zu gewährleisten, kann die Waage entweder zum homogenen Mischen mit einem Rüttler verbunden sein, der Eimer mit einem Rührsystem ausgestattet sein oder die Waage selbst eine integrierte Bewegungssteuerung aufweisen. Zum homogenen Mischen kann ferner eine separate Rüttelmaschine eingesetzt werden. In einer Rüttelmaschine kann ein fertig dosierter Eimer eingespannt und kräftig mittels Oszillations- und Rotationsbewegungen durchmischt werden.

An die Steuereinrichtung ist eine Tastatur oder dergleichen Eingabevorrichtung angeschlossen. Damit kann die Dosieranlage gesteuert werden und insbesondere die Dosierventile und die Förderpumpen für die Dispersionsfarbe oder Pigmentpaste entsprechend der gewünschten Mengen gesteuert werden.

Zudem kann an die Steuereinrichtung ein Drucker für ein an einem Eimer anzubringendes Etikett vorgesehen sein, mit dem die Daten gegebenenfalls auch maschinenlesbar, beispielsweise als Strichcode auf das Etikett gedruckt werden, um nach Aufkleben des Etiketts die in den Eimer abgefüllte Dispersionsfarbe an der Kasse abzurechnen.

Die erfindungsgemäße Dosieranlage eignet sich besonders gut als Mischanlage für Farben, auch "*Farbmischanlage*" genannt. Ein weiterer Aspekt der Erfindung betrifft daher eine Farbmischanlage mit einer Dosieranlage nach einem der Ansprüche 8 bis 13.

Bei einer Dosieranlage zum Mischen von Farben werden vorzugsweise mehrere Behälter miteinander über ein Steuersystem gesteuert, so dass verschiedene Dispersionsfarben oder Pigmentpasten miteinander kombiniert werden können. Beispielsweise können einige Behälter Dispersionen mit Füllstoffen und/oder Polymerdispersionen enthalten, während andere Behälter Dispersionen mit Pigment enthalten. Es ist aber auch möglich, dass ein Behälter bereits alle wesentlichen Komponenten für eine Dispersionsfarbe als Konzentrat umfasst und anschließend durch das Verdünnen mit Wasser zur Dispersionsfarbe wird. Ferner kann jeder der Behälter eine Pigmentpaste enthalten. Mit den Pigmentpasten in den verschiedenen Behältern kann eine Grundfarbe, beispielsweise eine weiße Dispersionsfarbe, bis zum Erreichen des gewünschten Farbtons abgetönt werden.

Gemäß einer bevorzugten Ausführungsform bestehen die Dispersionsfarben oder Pigmentpasten aus unterschiedlichen farbigen Pigmentpasten, die volumetrisch in einem vorgefertigten Basismaterial, welches sich als definierte Materialmenge bereits im Verkaufsbehälter befindet, zur Erreichung des gewünschten Farbtons kombiniert zugeführt werden.

Durch computergestützte Beratung und Produktauswahl ergibt sich eine Vielzahl von Kombinationsmöglichkeiten. Soll beispielsweise eine rote matte Innenfarbe angemischt werden, können mit der Eingabevorrichtung ein hoher Anteil an Pigmentrotpaste mit je nach Nuance geringeren Anteilen an Pigmentblaupaste und Pigmentschwarzpaste eingestellt werden. Zuvor wird mit der Eingabevorrichtung die Menge der sich im Eimer befindlichen Basisfarbe auf der Waage austariert. Über die Steuereinrichtung werden dann die Dosierventile und Förderpumpen entsprechend gesteuert, wobei bei Erreichen der vorgegebenen mit der Waage gemessenen Menge der in den Eimer zugeführten Pigmentpasten die Dosierventile geschlossen und die Förderpumpen abgeschaltet werden. Das gleiche Verfahren kann auch mit einer volumetrischen Dosierung durchgeführt werden, wobei in diesem Fall auf die Waage verzichtet wird.

Das im Zusammenhang mit dem erfindungsgemäßen Verfahren zum Oxidationsmittel und zur Einbringung des Oxidationsmittels Gesagte gilt gleichermaßen auch für die erfindungsgemäße Dosieranlage.

Das im Zusammenhang mit dem erfindungsgemäßen Verfahren zur Dispersionsfarbe oder Pigmentpaste Gesagte gilt gleichermaßen auch für die erfindungsgemäße Dosieranlage.

Die erfindungsgemäße Dosieranlage eignet sich insbesondere zur Durchführung des erfindungsgemäßen Verfahrens.

Nachstehend wird die Erfindung anhand der Zeichnungen näher erläutert, die jedoch nur der Veranschaulichung dienen und nicht limitierend sind.
- Fig. 1: zeigt eine Ausgestaltung der Dosieranlage gemäß der Erfindung,
- Fig. 2: zeigt einen Behälter im Querschnitt gemäß einer Ausführungsform der erfindungsgemäßen Dosieranlage mit einem zentralen Ozongenerator,
- Fig. 3: zeigt einen Behälter im Querschnitt gemäß einer Ausführungsform der erfindungsgemäßen Dosieranlage mit Ozonerzeugung im Deckel des Behälters,
- Fig. 4: zeigt einen Behälter im Querschnitt gemäß einer Ausführungsform der erfindungsgemäßen Dosieranlage mit einem zentralen Ozongenerator,
- Fig. 5: zeigt einen Behälter im Querschnitt gemäß einer Ausführungsform der erfindungsgemäßen Dosieranlage mit Ozonerzeugung im Deckel des Behälters,
- Fig. 6: zeigt einen Behälter im Querschnitt gemäß einer Ausführungsform der erfindungsgemäßen Dosieranlage mit einem zentralen Ozongenerator
- Fig. 7a: zeigt eine perspektivische Ansicht eines Adapters des Behälterdeckels gemäß einer Ausführungsform der Erfindung mit Ozonerzeugung in einem zentralen Ozongenerator,
- Fig. 7b: zeigt den Adapter des Behälterdeckels aus Fig. 7a im Querschnitt,
- Fig. 8a: zeigt eine perspektivische Ansicht eines Adapters des Behälterdeckels gemäß einer Ausführungsform der Erfindung mit Ozonerzeugung im Deckel des Behälters,
- Fig. 8b: zeigt den Adapter des Behälterdeckels aus Fig. 8a im Querschnitt.

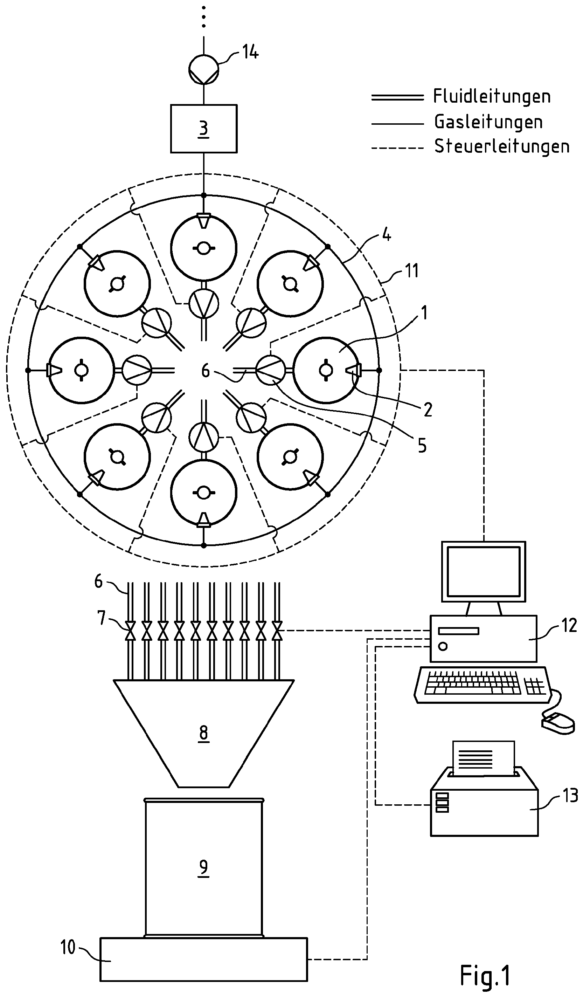

Fig. 1 zeigt eine bevorzugte Ausführungsform der erfindungsgemäßen Dosieranlage umfassend vorliegend mehrere Behälter 1. Die Behälter 1 weisen jeweils einen Höchstfüllstand für eine Dispersionsfarbe oder Pigmentpaste auf (in Fig. 1 nicht dargestellt). Jeder Behälter 1 weist zudem ein schließbares Dosierventil 7 sowie eine in den Behälter 1 führende Zuleitung für ein Oxidationsmittel auf, wobei jede Zuleitung von einer die einzelnen Zuleitungen speisenden Ringleitung 4 abgeht. Ringleitung 4 und die Zuleitungen sind aus Polyurethan gefertigt. Vorliegend sind acht zylinderförmige Behälter 1 aus Polyoxymethylen mit einem Volumen von je ein Liter karussellartig angeordnet, wobei das Ozon jeweils durch eine am Ende einer jeden Zuleitung angeordnete Düse 2 in den jeweiligen Behälter 1 eingebracht wird. Das Ozon wird zentral in einem Ozongenerator 3, umfassend eine Spannungsquelle, insbesondere einen Hochspannungsgenerator, und eine Entladungseinheit, mit einer Leistung von 200 mg Ozon pro Stunde erzeugt und gelangt, wie erwähnt, mit Hilfe einer Membranpumpe 14 über die Zuleitung durch die Düsen 2 in die zylinderförmigen Behälter 1. Dadurch kann in die Behälter 1 innerhalb von fünf Minuten jeweils etwa 2 mg Ozon eingebracht werden. Die in den zylinderförmigen Behältern 1 enthaltene Dispersionsfarbe oder Pigmentpaste wird über Förderpumpen 5 abgepumpt und entlang Förderleitungen 6 abgeführt. Die in die Förderleitungen 6 eingebauten Dosierventile 7 ermöglichen die genaue Dosierung der in den Behältern 1 enthaltenen Dispersionsfarbe oder Pigmentpaste. Die Dispersionsfarben oder Pigmentpasten werden jeweils nach Passieren des Dosierventils 7 über die Förderleitungen 6 zu einem Füllkopf 8 weiter geführt und gelangen von dort in einen Eimer 9. Der Eimer 9 steht dabei auf einer Waage 10. Die Förderpumpen 5, die Dosierventile 7 und die Waage 10 sind über die Steuerleitung 11 mit einem Computer 12 verbunden, über den eine exakte Dosierung der Dispersionsfarbe oder Pigmentpaste gesteuert wird. Der Computer 12 ist zudem mit einem Drucker 13 verbunden, der sich für das Drucken von Etiketten eignet.

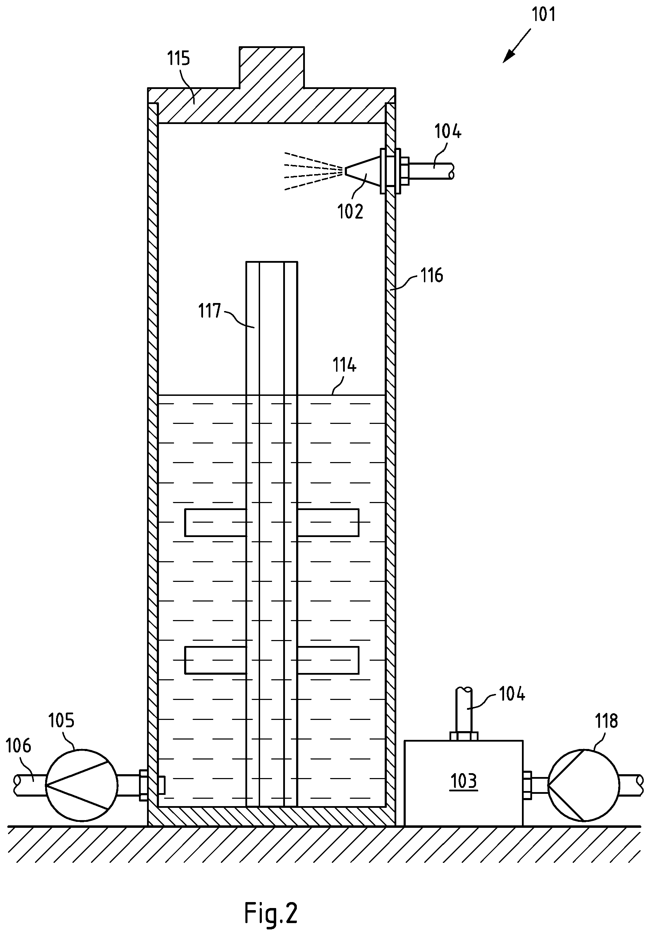

Fig. 2 zeigt einen Behälter im Längsschnitt gemäß einer Ausführungsform der erfindungsgemäßen Dosieranlage mit externem Ozongenerator. Im externen Ozongenerator 103, umfassend eine Spannungsquelle, insbesondere einen Hochspannungsgenerator, und eine Entladungseinheit, wird das Oxidationsmittel Ozon erzeugt und gelangt mit Hilfe einer Membranpumpe 118 über die Zuleitung 104 aus Polyurethan durch die Düse 102 in den zylinderförmigen Behälter 101 aus Polyoxymethylen in den Gasraum oberhalb der Oberfläche 114 einer im Behälter 101 befindlichen Dispersionsfarbe oder Pigmentpaste. Die Oberfläche 114 der Dispersionsfarbe oder Pigmentpaste fällt hier mit dem Höchstfüllstand des Behälters 101 zusammen. Der zylinderförmige Behälter 101 ist mit einem Deckel 115 und einer Seitenwand 116 ausgestattet. Die im Inneren des zylinderförmigen Behälters 101 befindliche Dispersionsfarbe oder Pigmentpaste wird durch das Rührwerk 117 vermischt. Die Dispersionsfarbe oder Pigmentpaste wird mithilfe der Förderpumpe 105 entlang der Förderleitung 106 aus dem Behälter 101 abgepumpt.

Fig. 3 zeigt einen Behälter 201 im Längsschnitt gemäß einer Ausführungsform der erfindungsgemäßen Dosieranlage mit Ozonerzeugung in einem Adapter 215b als Bestandteil des Behälterdeckels 218. Hierzu wird Luft, Sauerstoff oder ein Sauerstoff enthaltendes Gasgemisch, nachfolgend "Sauerstoffgasgemisch", durch die Öffnung 219 in den Innenraum des Adapters 215b eingebracht, in dem eine Entladungseinheit 203 angeordnet ist, die über den Hochspannunganschluss 220 an einen Hochspannungsgenerator, angeschlossen ist. Das in der Entladungseinheit entstehende Ozon/Luft, Ozon/Sauerstoff oder Ozon/Sauerstoffgasgemisch gelangt entlang der Zuleitung 221 in den Innenraum des zylinderförmigen Behälters 201 und dort in den Bereich oberhalb der Oberfläche 214 einer im Behälter 201 befindlichen Dispersionsfarbe oder Pigmentpaste. Der zylinderförmige Behälter 201 ist mit einem Deckel 218 umfassend Deckelteil 215a und, wie oben erwähnt, Adapter 215b sowie mit einer Seitenwand 216 ausgestattet. Die im Inneren des zylinderförmigen Behälters 201 befindliche Dispersionsfarbe oder Pigmentpaste wird durch das Rührwerk 217 vermischt. Die Dispersionsfarbe oder Pigmentpaste wird mithilfe der Förderpumpe 205 entlang der Förderleitung 206 aus dem Behälter 201 abgepumpt.

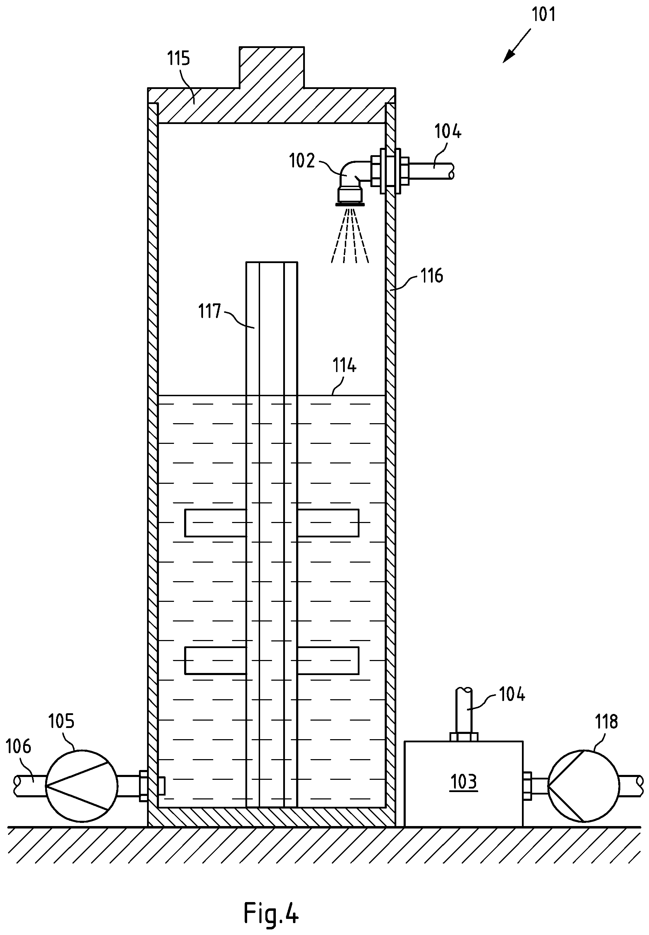

Fig. 4 zeigt einen Behälter im Längsschnitt gemäß einer Ausführungsform der erfindungsgemäßen Dosieranlage mit externem Ozongenerator. Im externen Ozongenerator 103, umfassend eine Spannungsquelle, insbesondere einen Hochspannungsgenerator, und eine Entladungseinheit, wird das Oxidationsmittel Ozon erzeugt und gelangt mit Hilfe einer Membranpumpe 118 über die Zuleitung 104 aus Polyurethan durch die Düse 102 in den zylinderförmigen Behälter 101 aus Polyoxymethylen in den Gasraum oberhalb der Oberfläche 114 einer im Behälter 101 befindlichen Dispersion. Die Düse 102 ist in Richtung des Behälterbodens gerichtet. Durch diese Düsenanordnung wird eine Verschmutzung der Düse 102 beim Befüllen des Behälters 101 mit der Dispersionsfarbe oder Pigmentpaste vermieden. Die Oberfläche 114 der Dispersionsfarbe oder Pigmentpaste fällt hier mit dem Höchstfüllstand des Behälters 101 zusammen. Der zylinderförmige Behälter 101 ist mit einem Deckel 115 und einer Seitenwand 116 ausgestattet. Die im Inneren des zylinderförmigen Behälters 101 befindliche Dispersionsfarbe oder Pigmentpaste wird durch das Rührwerk 117 vermischt. Die Dispersionsfarbe oder Pigmentpaste wird mithilfe der Förderpumpe 105 entlang der Förderleitung 106 aus dem Behälter 101 abgepumpt.

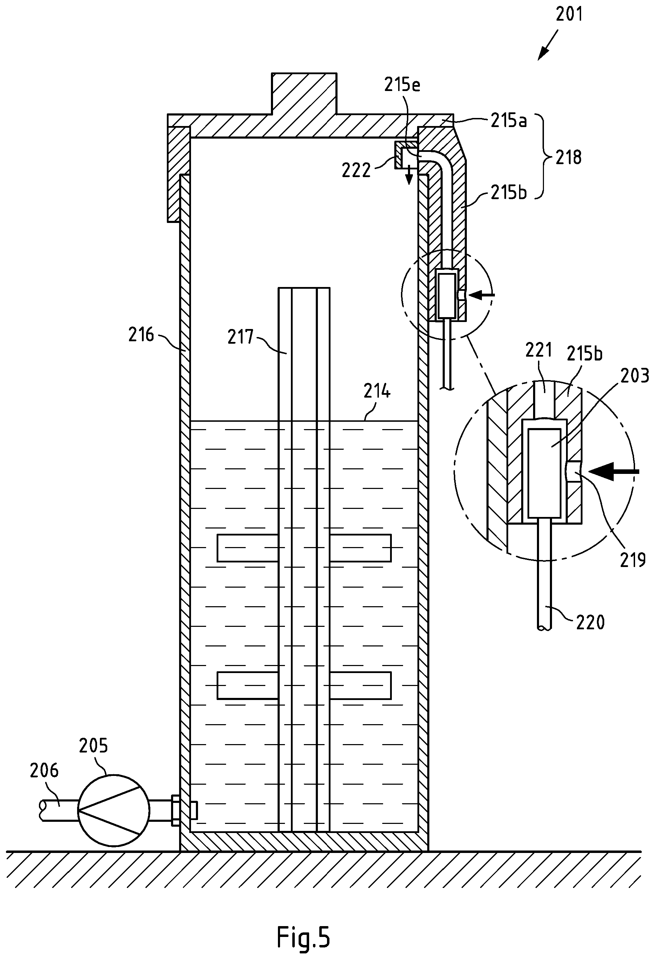

Fig. 5 zeigt einen Behälter 201 im Längsschnitt gemäß einer Ausführungsform der erfindungsgemäßen Dosieranlage mit Ozonerzeugung in einem Adapter 215b als Bestandteil des Behälterdeckels 218. Hierzu wird das Sauerstoffgasgemisch durch die Öffnung 219 in den Innenraum des Adapters 215b eingebracht, in dem eine Entladungseinheit 203 angeordnet ist, die über den Hochspannunganschluss 220 an einen Hochspannungsgenerator, angeschlossen ist. Das in der Entladungseinheit entstehende Ozon/Luft, Ozon/Sauerstoff oder Ozon/Sauerstoffgasgemisch gelangt entlang der Zuleitung 221 in den Innenraum des zylinderförmigen Behälters 201 und dort in den Bereich oberhalb der Oberfläche 214 einer im Behälter 201 befindlichen Dispersionsfarbe oder Pigmentpaste. An der Austrittsöffnung 215e der Zuleitung 221, durch die das Ozon/Luft, Ozon/Sauerstoff oder Ozon/Sauerstoffgasgemisch in den Innenraum des zylinderförmigen Behälters 201 gelangt, befindet sich eine nach unten geöffnete Schutzkappe 222. Durch die Schutzkappe 222 wird eine Verschmutzung und ein daraus resultierendes Verstopfen der Austrittsöffnung 215e beim Befüllen des Behälters 101 mit Dispersionsfarbe oder Pigmentpaste vermieden. Der zylinderförmige Behälter 201 ist mit einem Deckel 218 umfassend ein Deckelteil 215a und, wie oben erwähnt, Adapter 215b sowie mit einer Seitenwand 216 ausgestattet. Die im Inneren des zylinderförmigen Behälters 201 befindliche Dispersionsfarbe oder Pigmentpaste wird durch das Rührwerk 217 vermischt. Die Dispersionsfarbe oder Pigmentpaste wird mithilfe der Förderpumpe 205 entlang der Förderleitung 206 aus dem Behälter 201 abgepumpt.

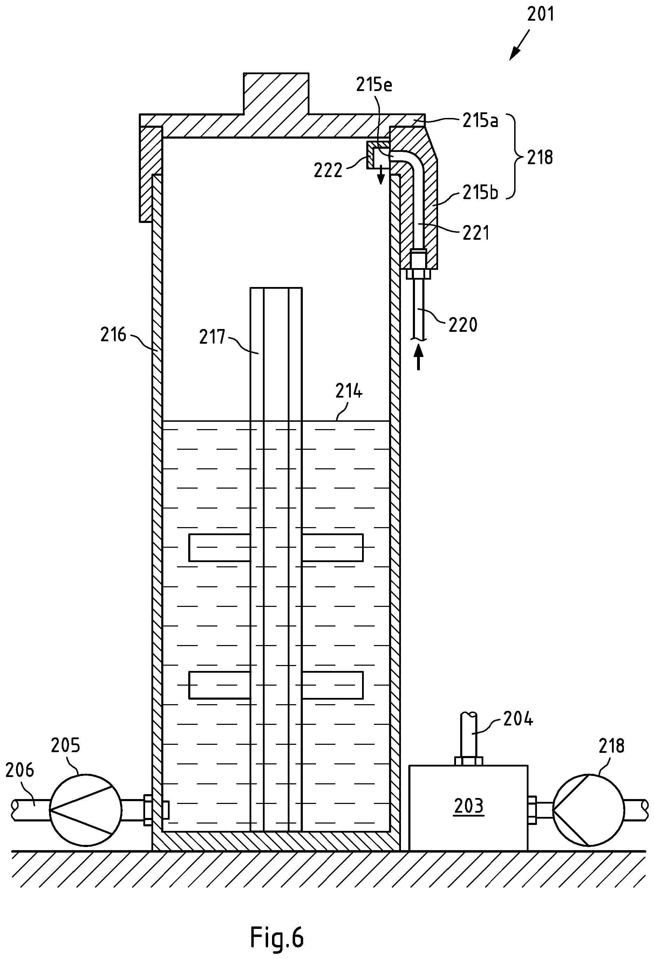

Fig. 6 zeigt einen Behälter im Längsschnitt gemäß einer Ausführungsform der erfindungsgemäßen Dosieranlage mit externem Ozongenerator. Die Einleitung des Ozons in den Innenraum des Behälters 201 erfolgt über einem Adapter 215b als Bestandteil des Behälterdeckels 218. Im externen Ozongenerator 203, umfassend eine Spannungsquelle, insbesondere einen Hochspannungsgenerator, und eine Entladungseinheit, wird das Oxidationsmittel Ozon erzeugt und gelangt mit Hilfe einer Membranpumpe 218 über die Zuleitung 204 aus Polyurethan zum Adapter 215b. Die Zuleitung 204 wird über einen Anschlussstutzen 223 mit dem Adapter 215b verbunden. Das Ozon gelangt durch die Zuleitung 221 in den zylinderförmigen Behälter 201 aus Polyoxymethylen in den Gasraum oberhalb der Oberfläche 214 einer im Behälter 201 befindlichen Dispersionsfarbe oder Pigmentpaste. An der Austrittsöffnung 215e der Zuleitung 221, durch die das Ozon in den Innenraum des zylinderförmigen Behälters 201 gelangt, befindet sich eine nach unten geöffnete Schutzkappe 222. Durch die Schutzkappe 222 wird eine Verschmutzung und ein daraus resultierendes Verstopfen der Austrittsöffnung 215e beim Befüllen des Behälters 101 mit Dispersionsfarbe oder Pigmentpaste vermieden. Der zylinderförmige Behälter 201 ist mit einem Deckel 218 umfassend ein Deckelteil 215a und, wie oben erwähnt, Adapter 215b sowie mit einer Seitenwand 216 ausgestattet. Die im Inneren des zylinderförmigen Behälters 201 befindliche

Dispersionsfarbe oder Pigmentpaste wird durch das Rührwerk 217 vermischt. Die Dispersionsfarbe oder Pigmentpaste wird mithilfe der Förderpumpe 205 entlang der Förderleitung 206 aus dem Behälter 201 abgepumpt.

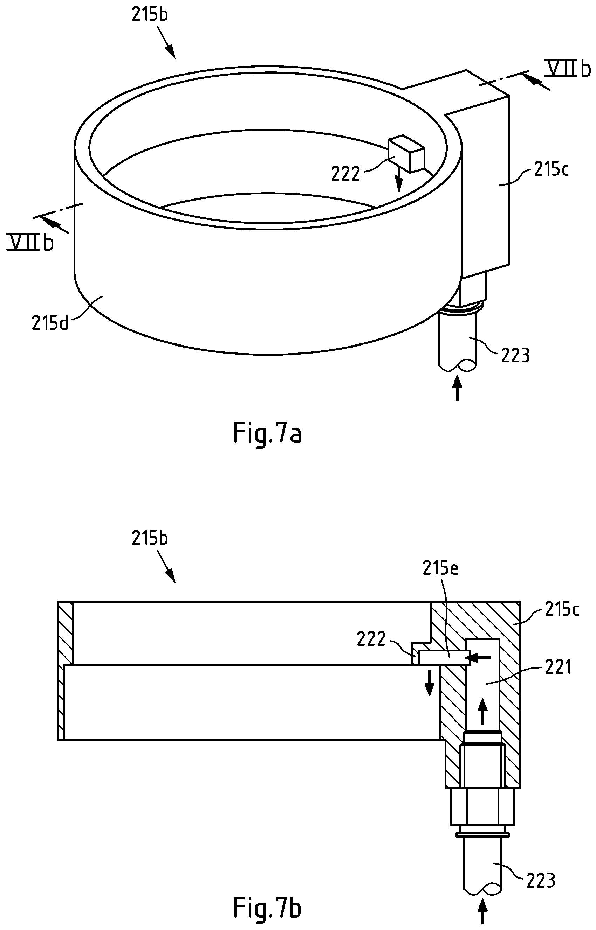

Fig. 7a und 7b zeigen einen Adapter 215b zum Einsatz in einer erfindungsgemäßen Dosieranlage mit externem Ozongenerator. Der Adapter 215b umfasst einen Anschlussabschnitt 215c und einen damit verbundenen ringförmigen Abschnitt 215d. Der ringförmige Abschnitt 215d ist so ausgestaltet, dass er an seiner Oberseite mit dem Deckelteil 215a und an seiner Unterseite mit der Seitenwand des Behälters 201 im Wesentlichen dicht abschließt. Der Anschlussabschnitt 215c weist einen Anschlussstutzen 223 für eine Gaszuleitung auf, über die der Adapter 215b an die Ozonzuleitung 204 angeschlossen werden kann. Der Anschlussabschnitt 215c umfasst eine Zuleitung 221, über die das Ozon durch die Austrittsöffnung 215e in den vom ringförmigen Abschnitt umgrenzten Innenraum geleitet wird. An der Austrittsöffnung 215e befindet sich eine nach unten geöffnete Schutzkappe 222, die verhindert, dass beim Befüllen des Behälters 201 Dispersionsfarbe oder Pigmentpaste in die Austrittsöffnung gelangt.

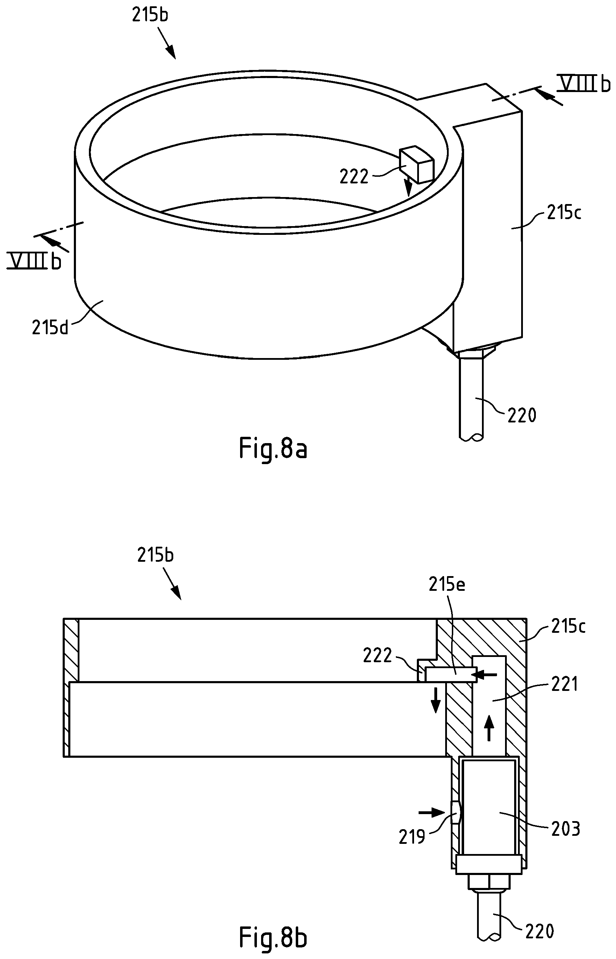

Fig. 8a und 8b zeigen einen Adapter 215b zum Einsatz in einer erfindungsgemäßen Dosieranlage mit Ozonerzeugung im Adapter 215b als Bestandteil des Behälterdeckels 218. Der Adapter 215b umfasst einen Anschlussabschnitt 215c und einen damit verbundenen ringförmigen Abschntt 215d. Der ringförmige Abschnitt 215d ist so ausgestaltet, dass er an seiner Oberseite mit dem Deckelteil 215a und an seiner Unterseite mit der Seitenwand des Behälters 201 im Wesentlichen dicht abschließt. Im Inneren des Anschlussabschnitts 215c ist eine Entladungseinheit 203 angeordnet, die über den Hochspannunganschluss 220 an einen Hochspannungsgenerator, angeschlossen ist. Der Anschlussabschnitt 215c umfasst eine Zuleitung 221, über die das in der Entladungseinheit gebildete Ozon/Luft, Ozon/Sauerstoff oder Ozon/Sauerstoffgasgemisch durch die Austrittsöffnung 215e in den vom ringförmigen Abschnitt umgrenzten Innenraum geleitet wird. An der Austrittsöffnung 215e befindet sich eine nach unten geöffnete Schutzkappe 222, die verhindert, dass beim Befüllen des Behälters 201 Dispersionsfarbe oder Pigmentpaste in die Austrittsöffnung gelangt.

Die in den Figuren 2 bis 8 abgebildeten Behälter und Adapter sind für den Betrieb mit gasförmigen Oxidationsmitteln wie z.B. Ozon geeignet. Sie können jedoch auch mit beliebigen anderen hierin beschriebenen Oxidationsmitteln, insbesondere mit flüssigen Oxidationsmitteln, betrieben werden. Die in den Figurenbeschreibungen enthaltenen Materialangaben (wie beispielsweise Polyoxymethylen für den Behälter oder Polyurethan für die Zuleitung) sind für die Ausführung der Erfindung nicht wesentlich und können durch beliebige andere geeignete Materialien, insbesondere die hierin beschriebenen Materialien, ersetzt werden. Auch die in den Figurenbeschreibungen enthaltenen Formangaben (wie beispielsweise zylinderförmiger Behälter) sind nicht wesentlich für die Ausführung der Erfindung und können durch beliebige andere geeignete Formen ersetzt werden (wie beispielsweise quaderförmige Behälter etc.). Ebensowenig sind beispielsweise die in den Figurenbeschreibungen angegebene genaue Anzahl und Position der Zuleitungen des Oxidationsmittels im Inneren des Behälters oder des Adapters wesentlich für die Ausführung der Erfindung und können, insbesondere wie hierin beschrieben, entsprechend variiert werden.

### Bezugszeichenliste

- 1: Behälter
- 2: Düse
- 3: Ozongenerator
- 4: Zuleitung
- 5: Förderpumpe
- 6: Förderleitung
- 7: Dosierventil
- 8: Füllkopf
- 9: Eimer
- 10: Waage
- 11: Steuerleitungen
- 12: Computer
- 13: Drucker
- 14: Membranpumpe

- 101: Behälter
- 102: Düse
- 103: Ozongenerator
- 104: Zuleitung
- 105: Förderpumpe
- 106: Förderleitung
- 114: Oberfläche der Dispersion
- 115: Deckel
- 116: Seitenwand
- 117: Rührwerk
- 118: Membranpumpe
- 201: Behälter
- 203: Entladungseinheit
- 205: Förderpumpe
- 206: Förderleitung
- 214: Oberfläche der Dispersion
- 215a: Deckelteil
- 215b: Adapter
- 215c: Anschlussabschnitt
- 215d: ringförmiger Abschnitt
- 215e: Austrittsöffnung
- 216: Seitenwand
- 217: Rührwerk
- 218: Behälterdeckel
- 219: Öffnung
- 220: Hochspannungsanschluss
- 221: Zuleitung
- 222: Schutzkappe
- 223: Anschlussstutzen

## Patentansprüche

1. Verfahren zur Konservierung einer Dispersionsfarbe oder einer Pigmentpaste in einer Dosieranlage, wobei die Dispersionsfarbe oder Pigmentpaste in einem Behälter (1, 101, 201) gelagert wird, welcher Teil der Dosieranlage ist, bei dem ein gasförmiges Oxidationsmittel in den Behälter (1, 101, 201) eingebracht wird, **dadurch gekennzeichnet, dass** das gasförmige Oxidationsmittel in den Gasraum über der Oberfläche (114, 214) der Dispersionsfarbe oder Pigmentpaste in den Behälter (1, 101, 201) eingebracht wird.

2. Verfahren nach Anspruch 1, **dadurch gekennzeichnet, dass** das Oxidationsmittel in periodischen Zeitabständen, insbesondere mindestens einmal pro Monat oder mindestens zweimal pro Monat oder mindestens dreimal pro Monat oder mindestens einmal pro Woche oder mindestens einmal pro Tag, in den Behälter eingebracht wird, und/oder **dadurch gekennzeichnet, dass** pro Einbringung 0,1 bis 200 mg, insbesondere 0,5 bis 100 mg oder 1 bis 50 mg, gerechnet pro Liter Behältervolumen, des Oxidationsmittels eingebracht werden.

3. Verfahren nach einem der vorstehenden Ansprüche, **dadurch gekennzeichnet, dass** das die Dispersionsfarbe oder Pigmentpaste nach dem Einbringen des Oxidationsmittels durchmischt wird, insbesondere durch Rühren.

4. Verfahren nach einem der vorstehenden Ansprüche, **dadurch gekennzeichnet, dass** das Oxidationsmittel mit Bezug auf die Normalwasserstoffelektrode bei einer Temperatur von 25°C und einer effektiven Konzentration von 1 mol l⁻¹ und/oder einer Ionenaktivität von 1 oder bei gasförmigen Reaktanden bei einem Partialdruck von 101,325 kPa ein Normalpotential von 0,1 V oder höher, bevorzugt 0,5 V oder höher, weiter bevorzugt 1 V oder höher, aufweist.

5. Verfahren nach einem der vorstehenden Ansprüche, **dadurch gekennzeichnet, dass** das Oxidationsmittel ein sauerstoff- oder chlorbasiertes Oxidationsmittel oder eine Mischung davon ist, insbesondere ausgewählt ist aus der Gruppe, bestehend aus Natriumhypochlorit, Kaliumhypochlorit, Javelwasser, Chlor, Wasserstoffperoxid, Ozon, Peroxyessigsäure, Perborat, Percarbonat und Mischungen davon.

6. Verfahren nach einem der vorstehenden Ansprüche, **dadurch gekennzeichnet, dass** das Oxidationsmittel Ozon ist, wobei optional das Ozon zentral in der Dosieranlage oder im Deckel (218) des mindestens einen Behälters (201), insbesondere mittels Koronaentladung, erzeugt wird.

7. Verfahren nach einem der vorstehenden Ansprüche, **dadurch gekennzeichnet, dass** die Dispersionsfarbe oder Pigmentpaste vor dem Einbringen des Oxidationsmittels im Wesentlichen frei von Konservierungsmitteln ist.

8. Dosieranlage zur Konservierung einer Dispersionsfarbe oder Pigmentpaste umfassend mindestens einen Behälter (1, 101, 201), der zur Lagerung einer Dispersionsfarbe oder Pigmentpaste ausgelegt ist und einen Höchstfüllstand (114, 214) für die Dispersionsfarbe oder Pigmentpaste, ein schließbares Dosierventil (7) sowie mindestens eine in den Behälter (1, 101, 201) führende Zuleitung (4, 104, 221) für ein gasförmiges Oxidationsmittel aufweist, **dadurch gekennzeichnet, dass** die in den Behälter (1, 101, 201) führende Zuleitung (104, 221) oberhalb seines Höchstfüllstands (114, 214) in den Behälter (1, 101, 201) führt.

9. Dosieranlage nach Anspruch 8, **dadurch gekennzeichnet, dass** der Behälter (1, 101, 201) Mittel zum Durchmischen (117, 217) einer Dispersionsfarbe oder Pigmentpaste, insbesondere ein Rührwerk, aufweist.

10. Dosieranlage nach einem der Ansprüche 8 oder 9, **dadurch gekennzeichnet, dass** die in den Behälter (1, 101, 201) führende Zuleitung (104, 221) in einer Düse (102) endet.

11. Dosieranlage nach einem der Ansprüche 8 bis 10, **dadurch gekennzeichnet, dass** der mindestens eine Behälter mehr als eine in den Behälter führende Zuleitung aufweist, wobei die Zuleitungen auf derselben Höhe in den Behälter führen oder auf verschiedenen Höhen in den Behälter führen, vorzugsweise oberhalb und unterhalb des Höchstfüllstands.

12. Dosieranlage nach einem der Ansprüche 8 bis 11, **dadurch gekennzeichnet, dass** der mindestens eine Behälter (1, 101, 201) aus Kunststoff oder Edelstahl, insbesondere aus Polyoxymethylen (POM), Polypropylen, Polyethylen, Polyethylenterehpthalat, Polyamid oder Mischungen oder Blends davon, ist, und/oder **dadurch gekennzeichnet, dass** die Zuleitung (104, 221) aus einem Material enthaltend einen Kunststoff ausgewählt aus der Gruppe bestehend aus Polyurethan (PUR), Polytetrafluoroethylen (PTFE), Perfluoralkoxy-Polymer (PFA), Polyvinylidenfluorid, Perfluorkatschuk, Ethylen-Tetrafluorethylen, Tetrafluorethylen-Hexafluorpropylen-Copolymer, Ethylen-Chlortrifluorethylen, Ethylen-Propylen-Dien-Kautschuk, und Mischungen davon ist.

13. Dosieranlage nach einem der Ansprüche 8 bis 12, **dadurch gekennzeichnet, dass** die Dosieranlage einen Ozongenerator (3, 103) umfasst, der insbesondere eine Spannungsquelle, insbesondere einen Hochspannungsgenerator, und eine Entladungseinheit (203) umfasst, die insbesondere mittels Koronaentladung Ozon erzeugt, wobei optional die Spannungsquelle, insbesondere der Hochspannungsgenerator, und die Entladungseinheit (203) nicht im selben Gerätegehäuse angeordnet sind, wobei insbesondere die Entladungseinheit (203) in einem Adapter (215b) des Behälterdeckels (218) ist.

14. Farbmischanlage mit einer Dosieranlage nach einem der Ansprüche 8 bis 13.

## Claims

1. Process for preserving an emulsion paint or a pigment paste in a metering apparatus, the emulsion paint or pigment paste being stored in a container (1, 101, 201) which is part of the metering apparatus, in which a gaseous oxidant is introduced into the container (1, 101, 201), **characterized in that** the gaseous oxidant is introduced into the gas space above the surface (114, 214) of the emulsion paint or pigment paste into the container (1, 101, 201).

2. Process according to claim 1, **characterized in that** the oxidant is introduced into the container at periodic intervals, in particular at least once a month or at least twice a month or at least three times a month or at least once a week, or at least once a day, and/or **characterized in that** 0.1 to 200 mg, in particular 0.5 to 100 mg or 1 to 50 mg, calculated per liter of container volume, of the oxidant is introduced per introduction.

3. Process according to either of the preceding claims, **characterized in that** the emulsion paint or pigment paste is mixed after the introduction of the oxidant, in particular by stirring.

4. Process according to any of the preceding claims, **characterized in that** the oxidant has a standard potential of 0.1 V or higher, preferably 0.5 V or higher, more preferably 1 V or higher with respect to the standard hydrogen electrode at a temperature of 25°C and at an effective concentration of 1 mol l⁻¹ and/or at an ion activity of 1 or, in the case of gaseous reactants, at a partial pressure of 101.325 kPa.

5. Process according to any of the preceding claims, **characterized in that** the oxidant is an oxygen- or chlorine-based oxidant or a mixture thereof, in particular is selected from the group consisting of sodium hypochlorite, potassium hypochlorite, Javel water, chlorine, hydrogen peroxide, ozone, peroxyacetic acid, perborate, percarbonate, and mixtures thereof.

6. Process according to any of the preceding claims, **characterized in that** the oxidant is ozone, the ozone optionally being generated centrally in the metering apparatus or in the lid (218) of the at least one container (201), in particular by means of corona discharge.

7. Process according to any of the preceding claims, **characterized in that** the emulsion paint or pigment paste is substantially free of preservatives before the introduction of the oxidant.

8. Metering apparatus for preserving an emulsion paint or pigment paste, comprising at least one container (1, 101, 201) designed for storing an emulsion paint or pigment paste and having a maximum fill level (114, 214) for the emulsion paint or pigment paste, a closable metering valve (7) and at least one supply line (4, 104, 221) for a gaseous oxidant leading into the container (1, 101, 201), **characterized in that** the supply line (104, 221) leading into the container (1, 101, 201) leads into the container (1, 101, 201) above its maximum fill level (114, 214).

9. Metering apparatus according to claim 8, **characterized in that** the container (1, 101, 201) has means for mixing (117, 217) an emulsion paint or pigment paste, in particular an agitator.

10. Metering apparatus according to either of claims 8 or 9,
**characterized in that** the supply line (104, 221) leading into the container (1, 101, 201) ends in a nozzle (102).

11. Metering apparatus according to any of claims 8 to 10,
**characterized in that** the at least one container has more than one supply line leading into the container, the supply lines leading into the container at the same level or leading into the container at different levels, preferably above and below the maximum fill level.

12. Metering apparatus according to any of claims 8 to 11,
**characterized in that** the at least one container (1, 101, 201) is made of plastics material or stainless steel, in particular of polyoxymethylene (POM), polypropylene, polyethylene, polyethylene terephthalate, polyamide, or mixtures or blends thereof, and/or **characterized in that** the supply line (104, 221) is made of a material containing a plastics material selected from the group consisting of polyurethane (PUR), polytetrafluoroethylene (PTFE), perfluoroalkoxy polymer (PFA), polyvinylidene fluoride, perfluoro rubber, ethylene-tetrafluoroethylene, tetrafluoroethylene-hexafluoropropylene copolymer, ethylene-chlorotrifluoroethylene, ethylene-propylene-diene rubber, and mixtures thereof.

13. Metering apparatus according to any of claims 8 to 12,
**characterized in that** the metering apparatus comprises an ozone generator (3, 103) which, in particular, comprises a voltage source, in particular a high-voltage generator, and a discharge unit (203) which in particular generates ozone by means of corona discharge, the voltage source, in particular the high-voltage generator, and the discharge unit (203) optionally not being arranged in the same device housing, the discharge unit (203) in particular being in an adapter (215b) of the container lid (218).

14. Paint mixing apparatus comprising a metering apparatus according to any of claims 8 to 13.

## Revendications

1. Procédé permettant la conservation d'une peinture de dispersion ou d'une pâte pigmentaire dans une installation de dosage, dans lequel la peinture de dispersion ou la pâte pigmentaire est stockée dans un récipient (1, 101, 201) faisant partie de l'installation de dosage, dans lequel un agent d'oxydation gazeux est introduit dans le récipient (1, 101, 201), **caractérisé en ce que** l'agent d'oxydation gazeux est introduit dans de compartiment de gaz au-dessus de la surface (114, 214) de la peinture de dispersion ou de la pâte pigmentaire dans le récipient (1, 101, 201).

2. Procédé selon la revendication 1, **caractérisé en ce que** l'agent d'oxydation est introduit dans le récipient à intervalles de temps périodiques, en particulier au moins une fois par mois ou au moins deux fois par mois ou au moins trois fois par mois ou au moins une fois par semaine ou au moins une fois par jour, **et/ou caractérisé en ce que** 0,1 à 200 mg, en particulier 0,5 à 100 mg ou 1 à 50 mg, calculés par litre de volume de récipient, d'agent d'oxydation sont introduits par introduction.

3. Procédé selon l'une des revendications précédentes,
**caractérisé en ce que** la peinture de dispersion ou la pâte pigmentaire est mélangée, en particulier par agitation, après l'introduction de l'agent d'oxydation.

4. Procédé selon l'une des revendications précédentes,
**caractérisé en ce que** l'agent d'oxydation présente, par rapport à l'électrode normale à hydrogène, un potentiel normal de 0,1 V ou plus, de préférence de 0,5 V ou plus, plus préférablement de 1 V ou plus, à une température de 25 °C et à une concentration effective de 1 mol l⁻¹ et/ou à une activité ionique de 1 ou, en présence de réactifs gazeux, à une pression partielle de 101,325 kPa.

5. Procédé selon l'une des revendications précédentes, **caractérisé en ce que** l'agent d'oxydation est un agent d'oxydation à base d'oxygène ou de chlore ou un mélange de ceux-ci, en particulier choisi dans le groupe constitué d'hypochlorite de sodium, hypochlorite de potassium, eau de Javel, chlore, peroxyde d'hydrogène, ozone, acide peroxyacétique, perborate, percarbonate et mélanges de ceux-ci.

6. Procédé selon l'une des revendications précédentes,
**caractérisé en ce que** l'agent d'oxydation est de l'ozone, dans lequel l'ozone est éventuellement produit de manière centrale dans l'installation de dosage ou dans l'élément de recouvrement (218) de l'au moins un récipient (201), en particulier au moyen d'un effet corona.

7. Procédé selon l'une des revendications précédentes,
**caractérisé en ce que** la peinture de dispersion ou la pâte pigmentaire est sensiblement exempte de conservateurs avant l'introduction de l'agent d'oxydation.

8. Installation de dosage pour la conservation d'une peinture de dispersion ou d'une pâte pigmentaire, comprenant au moins un récipient (1, 101, 201), conçu pour le stockage d'une peinture de dispersion ou d'une pâte pigmentaire, et un niveau de remplissage maximal (114, 214) pour la peinture de dispersion ou la pâte pigmentaire, une soupape de dosage (7) pouvant être fermée, ainsi qu'au moins une conduite d'alimentation (4, 104, 221) menant dans le récipient (1, 101, 201) pour un agent d'oxydation gazeux, **caractérisée en ce que** la conduite d'alimentation (104, 221) menant dans le récipient (1, 101, 201) mène dans le récipient (1, 101, 201), au-dessus de son niveau de remplissage maximal (114, 214).

9. Installation de dosage selon la revendication 8, **caractérisée en ce que** le récipient (1, 101, 201) présente des moyens pour le mélange (117, 217) d'une peinture de dispersion ou d'une pâte pigmentaire, en particulier un agitateur.

10. Installation de dosage selon l'une des revendications 8 ou 9,
**caractérisée en ce que** la conduite d'alimentation (104, 221) menant dans le récipient (1, 101, 201) se termine par une buse (102).

11. Installation de dosage selon l'une des revendications 8 à 10, **caractérisée en ce que** l'au moins un récipient présente plus d'une conduite d'alimentation menant dans le récipient, dans laquelle les conduites d'alimentation mènent dans le récipient à la même hauteur ou mènent dans le récipient à des hauteurs différentes, de préférence au-dessus et en dessous du niveau de remplissage maximal.

12. Installation de dosage selon l'une des revendications 8 à 11, **caractérisée en ce que** l'au moins un récipient (1, 101, 201) est en matière plastique ou en acier inoxydable, en particulier en polyoxyméthylène (POM), polypropylène, polyéthylène, polyéthylène téréphtalate, polyamide ou mélanges ou mixtures de ceux-ci, **et/ou caractérisée en ce que** la conduite d'alimentation (104, 221) est en un matériau contenant une matière plastique choisie dans le groupe constitué de polyuréthane (PUR),
polytétrafluoroéthylène (PTFE), polymère de perfluoroalcoxy (PFA), fluorure de polyvinylidène, caoutchouc perfluoré, éthylène-tétrafluoroéthylène, copolymère tétrafluoroéthylène-hexafluoropropylène, éthylène-chlorotrifluoroéthylène, caoutchouc éthylène-propylène-diène, et mélanges de ceux-ci.

13. Installation de dosage selon l'une des revendications 8 à 12, **caractérisée en ce que** l'installation de dosage comprend un générateur d'ozone (3, 103) comprenant en particulier une source de tension, en particulier un générateur haute tension, et une unité de décharge (203) générant de l'ozone, en particulier au moyen d'un effet corona, dans laquelle la source de tension, en particulier le générateur haute tension, et l'unité de décharge (203) ne sont éventuellement pas disposées dans le même boîtier d'appareil, dans laquelle l'unité de décharge (203) est en particulier dans un adaptateur (215b) de l'élément de recouvrement de récipient (218).

14. Installation de mélange de couleurs comportant une installation de dosage selon l'une des revendications 8 à 13.
